# FASCICULE DE BREVET EUROPEEN

(11) **EP 0 653 225 B1**
(45) Date de publication et mention de la délivrance du brevet: **25.07.2001**
(21) Numéro de dépôt: 94402590.7
(22) Date de dépôt: 16.11.1994
(51) Int. Cl.: A61N 1/37

(54) **Procédé de commande automatique du seuil de détection de signaux cardiaques dans un appareil implantable**
Verfahren zur automatischen Steuerung der Herzsignalschwellenwerte bei einer implantierbaren Vorrichtung
Automatic threshold detection of cardiac signals for use in an implantable device

(30) Priorité: 17.11.1993 FR 9313733
(43) Date de publication de la demande: 17.05.1995
(73) Titulaire: ELA MEDICAL (Société anonyme), F-92541 Montrouge (FR)
(72) Inventeur: Jacobson, Peter, F-67500 Haguenau (FR); Kroiss, Daniel, F-67500 Scheweiehouse/Moder (FR)
(74) Mandataire: Dupuis-Latour, Dominique

(56) Documents cités:
- EP-A- 0 334 618
- EP-A- 0 399 059
- EP-A- 0 429 025
- EP-A- 0 549 438
- GB-A- 2 195 538

## Description

La présente invention concerne les circuits et les logiciels de détection de signaux cardiaques, notamment ceux des appareils implantables.

L'invention sera principalement décrite dans le contexte d'un appareil implantable tel qu'un cardioverteur ou un défibrillateur, destiné à traiter les tachycardies et les tachyarythmies, mais elle peut être également appliquée à des appareils tels que les stimulateurs cardiaques, destinés à traiter les bradycardies, ces deux catégories pouvant d'ailleurs être combinées en un seul et même appareil.

Plus précisément, l'invention concerne les circuits et logiciels de détection des dépolarisations cardiaques dans les tissus au voisinage d'une ou plusieurs électrodes en contact avec le myocarde ou à proximité de celui-ci en vue de permettre la détermination et l'analyse du rythme cardiaque (auriculaire et/ou ventriculaire) d'un patient en vue d'un traitement approprié : stimulation, cardioversion et/ou défibrillation.

Le problème particulier auquel s'attache l'invention est celui de l'ajustement automatique du seuil de sensibilité à un niveau suffisamment faible pour assurer une détection certaine de toutes les dépolarisations cardiaques, et suffisamment élevé pour éviter toute détection sur du bruit ou autres signaux parasites.

Ce problème a déjà été abordé par de nombreux auteurs.

Ainsi, le US-A-4 184 493 (Langer) décrit un circuit de détection pour défibrillateur implantable comprenant un amplificateur, un filtre passe-haut du premier ordre, un comparateur bidirectionnel et un circuit qui mesure le laps de temps pendant lequel le comparateur délivre un signal à sa sortie. Au bout du compte, le détecteur mesure le rapport moyen entre ECG de forte pente et ECG de faible pente. Le circuit ajuste automatiquement le gain de l'amplificateur afin de maintenir une amplitude constante en sortie du filtre.

Le US-A-4 393 877 (Imran) décrit un circuit de détection pour défibrillateur implantable qui comprend un circuit détecteur de pente et un circuit détecteur de passage à zéro. Le détecteur de pente comprend un amplificateur, un filtre passe-haut du premier ordre, un comparateur unidirectionnel et un multivibrateur monostable pour le séquencement d'une période réfractaire. Ici encore, le circuit ajuste automatiquement le gain de l'amplificateur afin de maintenir une amplitude constante en sortie du filtre.

Le US-A-4 659 946 (Mower) décrit un circuit de détection qui synchronise les chocs sur l'activité cardiaque dans un défibrillateur implantable, et qui comprend un circuit sensible à la pente suivi d'un comparateur. Le circuit ajuste automatiquement le seuil du comparateur à une fraction d'une valeur crête récente du signal de sortie du filtre.

Le US-A-4 619 643 (Menken) décrit un défibrillateur avec des fonctions de stimulation et de détection et un étage de détection de fibrillation distinct avec contrôle automatique de gain, qui retarde la stimulation jusqu'à ce que le gain de l'étage de détection de fibrillation atteigne une sensibilité maximale.

Le US-A-4 940 054 (Grevis) décrit un défibrillateur possédant au moins deux valeurs de sensibilité dont le choix dépend du type de rythme détecté.

Le US-A-4 880 004 (Baker) décrit un circuit de détection pour la détection des arythmies, qui comprend un amplificateur d'entrée suivi d'un étage de détection avec filtrage passe-haut à 25 Hz et un étage de mesure avec filtrage passe-haut supplémentaire à 12 Hz, chacun des étages étant suivi de comparateurs. Ce circuit ajuste automatiquement le gain de l'amplificateur afin de maintenir constante la marge de détection (quantité dont le signal de la voie de détection dépasse son seuil). Cette marge s'améliore pour les fréquences basses en raison du filtrage additionnel dans l'étage de mesure.

Le US-A-4 967 747 (Carroll) décrit un défibrillateur implantable avec un circuit de détection consistant en un bloc gain/filtrage à capacités commutées conçu de manière que le contrôle automatique de gain ne soit pas affecté par les pointes de conversion.

Le US-A-5 117 824 (Keimel) décrit un circuit implantable de stimulation et de détection avec un contrôle de seuil automatique qui n'ajuste pas le seuil après stimulation mais qui règle le seuil en fonction de l'amplitude mesurée après détection, en réduisant graduellement le seuil par la suite.

Le GB-A-2 195 538 décrit un circuit réjecteur de bruit pour un stimulateur. Dans ce circuit, après une période réfractaire absolue, la valeur du seuil de détection est abaissée à un niveau très bas (juste au-dessus du seuil de numérisation du convertisseur A/N), de manière à provoquer ensuite un déclenchement quasi-systématique du comparateur de détection pendant une première période réfractaire relative. Cette dernière est alors immédiatement redémarrée et le niveau de seuil réajusté, jusqu'à obtenir la réjection souhaitée du bruit parasite.

Cette question de l'ajustement de la sensibilité de détection, tout particulièrement (mais non exclusivement) dans les défibrillateurs et cardioverteurs, n'a pas encore reçu de réponse véritablement satisfaisante et est restée jusqu'à présent ouverte à de nombreuses controverses.

Ainsi, dans "State-of-the-Art of the AICD", *Pace*, mai 1991, Winkle décrit un défibrillateur sans contrôle automatique de gain, pour lequel "programmer la sensibilité nécessite un très grand soin au moment de l'implantation et lors du suivi pour être certain que les ondes T ne soient pas sur-détectées et, plus important encore, que la tachycardie/fibrillation ventriculaire ne soit pas sous-détectée".

Dans "Failure of a Second and Third Generation Implantable Cardioverter to Sense Ventricular Tachycardia: Implications for Fixed-Gain Sensing Devices", *Pace*, mai 1992, Sperry et coll. ont écrit que "des réglages à gain fixe plus sensibles ou une détection automatique du gain sont nécessaires pour détecter des signaux de faible amplitude de façon cohérente. L'aspect indésirable de l'utilisation d'une telle sensibilité élevée tient au fait que la sur-détection (par exemple d'ondes T) par l'appareil donne un risque accru de décharges à mauvais escient. Mais on a aussi déjà signalé que, avec des appareils à gain automatique, des fibrillations ventriculaires n'ont pas été détectées".

On voit ainsi qu'un circuit de détection approprié, tout particulièrement pour un détecteur de tachyarythmies, doit détecter les dépolarisations cardiaques mais rejeter les signaux indésirables, notamment et y compris la repolarisation cardiaque (onde T) et les signaux émanant du retour à l'équilibre du système d'électrodes et du circuit de sortie du stimulateur après stimulation, appelés "artefact de stimulation".

Les techniques de l'art antérieur évoquées plus haut utilisent des circuits de filtrage passe-haut, de comptage de périodes réfractaires et de contrôle automatique de gain (ou de seuil de détection). Mais les détecteurs de tachyarythmie de l'art antérieur ne prennent en compte que l'amplitude mesurée du signal pour régler leur sensibilité.

L'un des buts de la présente invention est de proposer un procédé de contrôle automatique du seuil de détection qui prenne en compte non seulement l'amplitude mesurée du signal, mais également l'amplitude du bruit pour ajuster et optimiser sa sensibilité, afin de régler le seuil de détection nettement au-dessous de l'amplitude du signal détecté mais au-dessus de l'amplitude du bruit et autres sources de parasites.

Un autre but de la présente invention est de proposer une commande automatique de seuil de détection qui, après stimulation, ajuste le seuil de détection à une valeur prédéterminée (c'est-à-dire une valeur ajustable par programmation externe et mémorisée une fois pour toutes par l'appareil), et accroît ce seuil de détection au-dessus de l'amplitude du bruit détecté après la stimulation.

Un autre but encore de la présente invention est de proposer une commande automatique de seuil de détection permettant d'éviter de modifier le seuil de détection lorsque des événements cardiaques isolés sont détectés au-delà de la durée d'une période réfractaire absolue après stimulation ou détection.

Le procédé de l'invention est du type connu enseigné par le GB-A-2 195 538 précité, correspondant au préambule de la revendication 1.

Pour atteindre les buts précité, l'invention propose de perfectionner ce procédé connu par les étapes énoncées dans la partie caractérisante de la revendication 1.

Les revendications 2 à 10 visent des mises en oeuvre avantageuses particulières de l'invention.

D'autres caractéristiques et avantages de l'invention apparaîtront à la lecture de la description d'un mode préférentiel de mise en oeuvre de l'invention, faite en référence aux dessins annexés.

La figure 1 est une vue générale d'un circuit de détection permettant de mettre en oeuvre le procédé de l'invention.

La figure 2 (scindée en deux figures 2a et 2b) est un organigramme donnant, de façon générale, le déroulement des différentes étapes de la forme préférentielle de mise en oeuvre du procédé de l'invention.

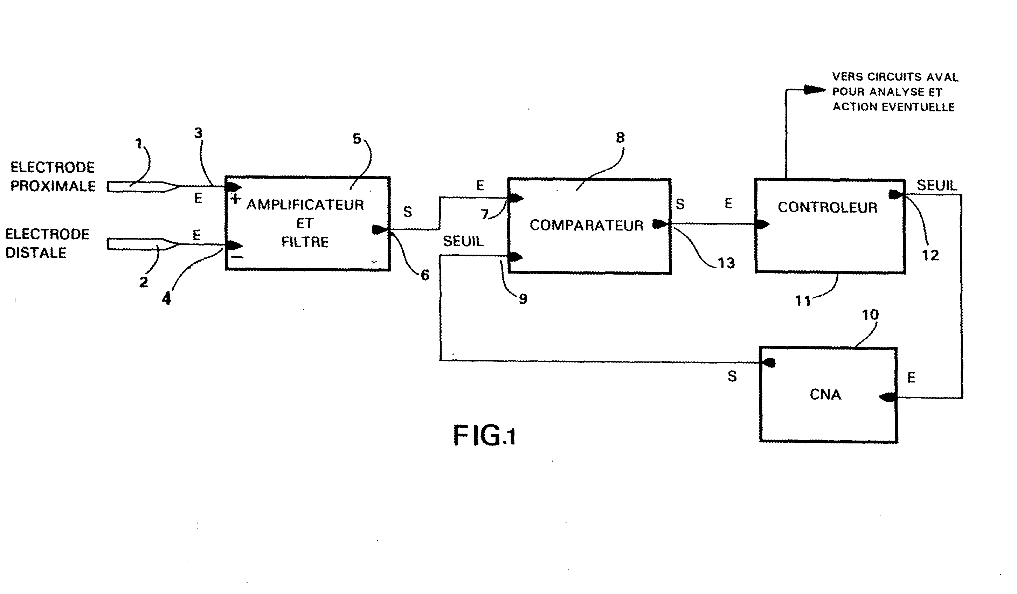

La figure 1 illustre sous forme de blocs fonctionnels un circuit de détection de signaux cardiaques utilisable avantageusement (mais non exclusivement) dans un appareil implantable de détection et de traitement des tachyarythmies.

Des électrodes proximale 1 et distale 2, placées directement sur le myocarde, amènent le signal de dépolarisation cardiaque aux entrées différentielles 3 et 4 d'un circuit 5 d'amplification et de filtrage (appelé ci-dessous simplement "filtre"). Dans la forme de réalisation préférée, ce filtre 5 présente une caractéristique passe-haut du troisième ordre avec une fréquence de coupure d'environ 16 Hz, au moins une caractéristique passe-bas du premier ordre avec une fréquence de coupure d'environ 100 Hz et un gain à mi-bande d'environ 125.

Le signal apparaissant en sortie 6 du filtre est appliqué à l'une des entrées 7 d'un comparateur 8, dont l'autre entrée 9 reçoit une tension de référence, que l'on appellera par la suite "seuil de détection". Cette tension de référence est de préférence produite par un convertisseur numérique/analogique 10 à partir de signaux produits par un contrôleur 11 sur la sortie 12 de ce dernier. Le contrôleur 11 est piloté en entrée par le signal apparaissant à la sortie 13 du comparateur 8, la présence de ce signal indiquant que le circuit a détecté une dépolarisation cardiaque. Le contrôleur transmet alors cette indication à des circuits situés en aval (non représentés, et connus en eux-mêmes) pour analyse du rythme cardiaque et déclenchement éventuel d'une action appropriée (stimulation, cardioversion et/ou défibrillation).

Le contrôleur 11 met en oeuvre diverses fonctions qui seront explicitées plus bas, notamment la gestion de périodes réfractaires et l'ajustement automatique et/ou programmé du seuil de détection, via le convertisseur 10.

À cet effet, le contrôleur 11 observe la sortie 13 du comparateur lorsqu'un signal est présent à la sortie 6 du filtre 5. Il peut alors évaluer l'amplitude du signal à cette sortie 6, car le comparateur 8 ne délivrera de signal à sa sortie 13 que lorsque le signal à son entrée 7 dépassera le seuil appliqué à l'entrée 9. Ceci peut être réalisé par approximations successives ou par génération d'une rampe de seuils, techniques bien connues dans le domaine des convertisseurs analogique/numérique et que l'on ne décrira pas plus en détail. On peut également mettre en oeuvre des convertisseurs "flash" consistant, selon un schéma également connu, en un ensemble de comparateurs ayant chacun son propre seuil et reliés chacun au contrôleur de manière que celui-ci puisse déterminer, à un moment donné, quels sont les seuils qui sont dépassés par le signal de sortie filtré de l'amplificateur.

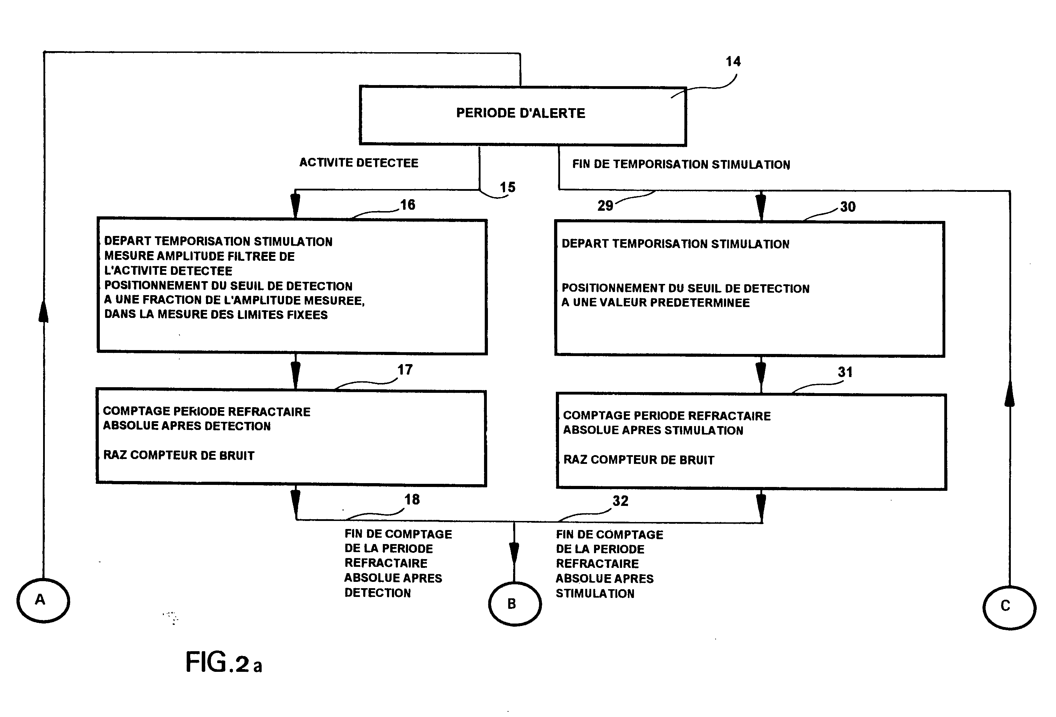

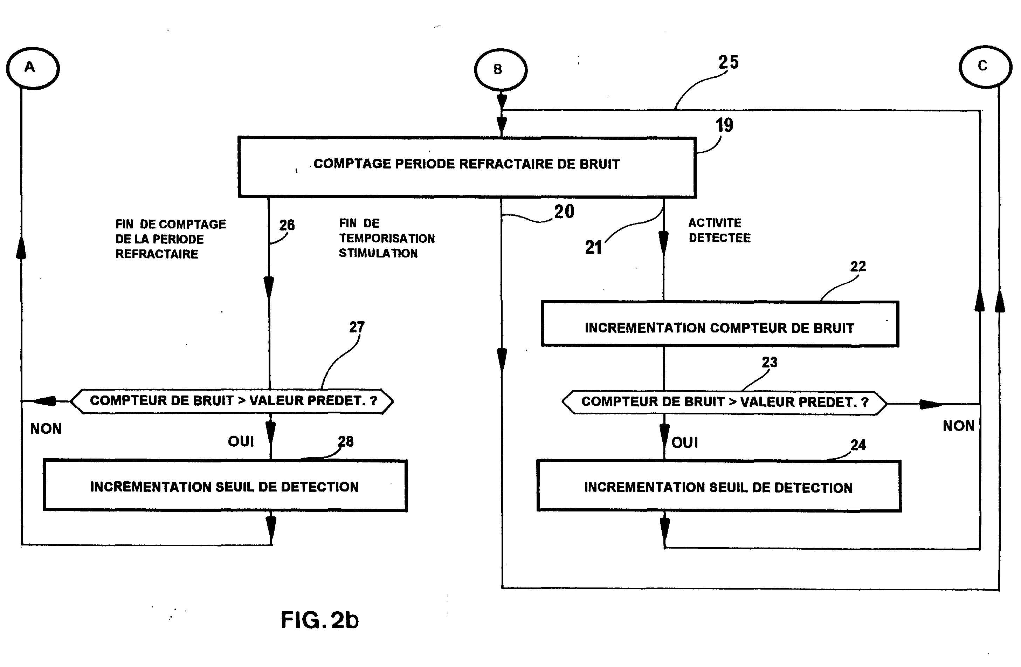

La figure 2 est un organigramme illustrant le mode préférentiel de mise en oeuvre du procédé de l'invention. Les diverses fonctions que l'on va décrire peuvent être implémentées par matériel ou par logiciel, ou par une combinaison de matériel et de logiciel, cette implémentation étant en elle-même à la portée du spécialiste de la technique.

En 14 sur la figure 2, on commence à compter une "période d'alerte", période au cours de laquelle on va attendre l'apparition d'une activité spontanée (branche 15) et, si aucune activité n'a été détectée (branche 29), on déclenchera systématiquement une stimulation en fin de période.

On considère qu'il y a activité détectée lorsque la sortie 6 du filtre 5 de la figure 1 dépasse le seuil de détection appliqué à l'entrée 9 du comparateur.

Pour déterminer la fin de la période d'alerte, on charge un compteur de temps après une stimulation ou après une détection survenues en dehors des périodes réfractaires. Le matériel et le logiciel de stimulation et de comptage des périodes sont de même nature que ceux utilisés dans les appareils de la technique antérieure et ne seront pas décrits plus en détail dans le cadre de la présente invention.

Toujours en référence à la figure 2, dans le cas référencé 15 de la détection d'une activité, on commence par mettre en oeuvre (étape 16) les fonctions illustrées sur la figure 1 afin de mesurer, de la manière décrite plus haut, l'amplitude filtrée de l'activité détectée. On règle ensuite le seuil de détection à une fraction prédéterminée de cette amplitude mesurée.

En pratique, de bons résultats ont été obtenus avec, pour cette fraction, une valeur allant d'un huitième à la moitié, et des performances optimales avec une valeur d'un quart.

À cet égard, on constate que l'amplitude des signaux cardiaques présente une large plage dynamique, variant d'un patient à l'autre et, pour un même patient, d'un moment à l'autre et en fonction de son état cardiaque, cette plage allant typiquement d'environ 0,4 mV jusqu'à plus de 20 mV (par commodité, dans la présente description, on se référera pour les amplitudes détectées et les seuils de détection à des valeurs équivalentes considérées en entrée du filtre 5).

On a aussi trouvé qu'il était avantageux d'accroître le seuil de détection au-dessus de 0,4 mV lorsque l'amplitude détectée dépassait quelques millivolts, afin d'éviter les détections multiples d'un même événement ; on a d'autre part constaté qu'un seuil plus bas, inférieur à environ 0,4 mV, accroissait la susceptibilité à la détection des événements en champ lointain, des bruits et des ondes T. Inversement, on a constaté que, si l'on prévoyait un seuil supérieur à environ 1,6 mV, on risquait d'omettre la détection de certains complexes de faible amplitude qui surviennent lors d'une fibrillation ventriculaire, même lorsque l'amplitude du battement précédent avait dépassé cette valeur d'un facteur quatre. Aussi, selon l'invention, après avoir réglé le seuil de détection à un quart de l'amplitude du dernier battement, on prévoit, si cette valeur donne un seuil inférieur à 0,4 mV, de forcer le seuil à 0,4 mV et, si cette valeur donne un seuil supérieur à 1,6 mV, de forcer ce seuil à 1,6 mV.

À l'étape 17, on commence à compter une "période réfractaire absolue après détection". Cette période sert à éviter des détections multiples du même événement, le contrôleur 11 restant inaffecté par l'activité cardiaque pendant la durée de cette période, typiquement d'environ 94 ms (cette valeur pouvant être ajustée par programmation externe). Toujours à l'étape 17, l'appareil remet à zéro (avant, pendant ou après la période réfractaire) un compteur appelé "compteur de bruit" dont on décrira plus bas le rôle.

En fin de période réfractaire absolue (branche 18), l'appareil commence à compter une "période réfractaire de bruit" (étape 19). Lors de cette période réfractaire de bruit, le contrôleur peut à nouveau détecter des événements, mais va considérer que ces événements sont du bruit, et ne déclenchera donc aucune stimulation en réponse. Si, avant la fin de cette période réfractaire de bruit (dont la durée peut être ajustée par programmation externe), on arrive (branche 20) en fin de comptage de la temporisation de stimulation démarrée à l'étape 16, on déclenche une stimulation du coeur (étape 30, *infra*). Dans ce cas, on peut aussi, en variante, omettre de stimuler le coeur si l'on se trouve dans un cycle où l'appareil continue à trouver du bruit au moment où il devrait stimuler, ceci afin d'éviter toute compétition avec un rythme cardiaque intrinsèque masqué par le bruit.

Lorsqu'une activité détectée apparaît (branche 21) avant la fin de la période réfractaire de bruit, l'appareil considère qu'il s'agit d'un bruit. On incrémente alors (étape 22) le compteur de bruit à chaque détection survenue pendant la durée de la période réfractaire de bruit. A l'étape 23, lorsque ce compteur dépasse une valeur prédéterminée, de préférence quatre comptages, l'appareil incrémente alors le seuil de détection (étape 24), typiquement de 0,2 mV. De cette manière, un nombre de détections de bruit supérieur à une valeur prédéterminée provoque une augmentation du seuil de détection jusqu'à ce que l'appareil ne détecte plus de bruit, ou jusqu'à ce que le seuil de détection atteigne une certaine valeur maximale à laquelle l'appareil ne détecte plus rien car cette valeur dépasse la plage dynamique du comparateur de détection.

Chaque fois que l'on détecte du bruit, on retourne (branche 25) à l'étape 19 pour réinitialiser et redémarrer la période réfractaire de bruit. Cette dernière va donc prendre fin soit lorsque le moment sera venu de stimuler (branche 20), comme expliqué plus haut, soit lorsque l'appareil n'aura rien détecté pendant une durée au moins égale à la durée prédéterminée de la période réfractaire de bruit (branche 26).

Dans ce dernier cas, si le compteur de bruit dépasse une valeur prédéterminée (étape 27), ceci signifie que l'appareil a détecté un certain nombre de fois une activité devant être considérée comme un bruit. Avant de continuer à compter la période d'alerte (étape 14) jusqu'à stimulation, on incrémente alors à nouveau le seuil de détection (étape 28), afin d'être sûr que ce seuil dépassera l'amplitude du bruit d'une certaine quantité minimale. Typiquement, un incrément de 0,2 mV à l'étape 28 suffit à empêcher toute détection ultérieure de bruit dans le même cycle cardiaque.

Le compteur de bruit incrémenté à l'étape 22 sert à éviter d'augmenter le seuil de détection dans le cas d'un événement cardiaque isolé de durée inhabituellement longue, afin que l'appareil puisse continuer à détecter l'événement même après la fin de la période réfractaire absolue.

Toutefois, on notera que l'utilisation du compteur de bruit n'est pas indispensable à la mise en oeuvre de l'invention dans la mesure où, pour de nombreux patients, la bande passante de l'amplificateur et la longueur de la période réfractaire absolue peuvent être des paramètres en eux-mêmes suffisants pour empêcher que des événements ne soient détectés en dehors de la période réfractaire absolue.

Avec les valeurs de bande passante et de période réfractaire indiquées plus haut, on a constaté que, en pratique, il n'était utile d'incrémenter le seuil de détection aux étapes 24 et 28 que lorsque le compteur de bruit dépassait une valeur prédéterminée de l'ordre de quatre comptages. En d'autres termes, on attend quatre détections d'un bruit avant de commencer à modifier le seuil de sensibilité. Ceci permet de ne pas modifier la sensibilité de détection dans les cas où le signal dure un peu plus longtemps que la période réfractaire absolue, ce qui est le cas de la détection éventuelle de l'onde T après une stimulation ventriculaire, mais également celui d'un complexe spontané qui durerait plus longtemps que la période réfractaire, ou encore celui d'un rythme à double foyer ou d'un rythme très rapide dont le couplage RR serait tel que la seconde onde R se situerait dans la fin de la période réfractaire absolue ou dans la période réfractaire de bruit. Si toutefois ce signal était du bruit, la sensibilité sera alors réajustée automatiquement à une valeur supérieure à l'amplitude mesurée, puisque ce signal se prolongerait.

En variante, l'appareil pourrait n'incrémenter le seuil de détection que pour les signaux détectés dans la période réfractaire de bruit, mais survenant après au moins une certaine durée prédéterminée suivant la fin de la période réfractaire absolue. On aboutirait à une fonction semblable à celle du compteur de bruit, en empêchant tout ajustement du seuil de détection d'un battement cardiaque isolé pendant un laps de temps dépassant la longueur de la période réfractaire absolue. Dans cette variante, on remplacerait la question de l'étape 27 par une question telle que : "Y a-t'il eu détection après une durée prédéterminée suivant la fin de la période réfractaire absolue ?".

Si l'on en revient maintenant au comptage de la période d'alerte (étape 14), si aucune activité n'est détectée et que l'on arrive (branche 29) en fin de comptage ― ce qui aura par ailleurs déclenché la stimulation ―, on ajuste alors le seuil de détection à une valeur prédéterminée (étape 30) et on commence à compter une "période réfractaire absolue après stimulation" (étape 31). La longueur de cette dernière peut dépasser notablement la durée de la période réfractaire absolue après détection comptée à l'étape 17, afin d'être sûr que l'appareil ne détecte pas l'artefact de stimulation. Typiquement, on choisit une période réfractaire absolue après stimulation supérieure à 175 ms. Toujours à l'étape 31, on remet à zéro le compteur de bruit.

Lorsque (branche 32) l'on a fini de compter la période réfractaire absolue après stimulation, on exécute les mêmes étapes de procédé (étapes 19 et s., décrites plus haut) que celles que l'on avait exécutées après la fin de comptage de la période réfractaire absolue après détection.

La durée de la période réfractaire de bruit découle du choix de la période maximale détectée en tant que bruit, à laquelle elle est égale. Mais ce choix résulte d'un compromis avec la période maximale détectée en tant que fibrillation et la période réfractaire absolue. En effet, afin d'assurer la détection en tant que fibrillation de tous les événements ventriculaires dont la période est comprise entre ladite période maximale détectée en tant que fibrillation et ladite période maximale détectée en tant que bruit, il est nécessaire que la somme de la période réfractaire absolue et de la période réfractaire de bruit soit inférieure, et à la limite égale, à la moitié de ladite période maximale détectée en tant que fibrillation. Par exemple, si la période minimale de détection de la fibrillation est programmée à 250 ms, avec une période réfractaire totale de 125 ms, tout rythme dont la période est inférieure à 250 ms et supérieure à la période réfractaire de bruit sera considéré comme une fibrillation.

On a constaté qu'une valeur de 31 ms constituait un bon compromis pour la longueur de la période réfractaire de bruit, bien que des valeurs pouvant atteindre 100 ms pouvaient donner des résultats corrects avec certains patients. Ces valeurs plus longues pourraient aider à détecter des sources de bruit de fréquence plus basse, comme celles des chemins de fer nationaux allemands, qui fonctionnent à 17 Hz.

La présente description ne donne pas de détails de mise en oeuvre du matériel de l'amplificateur de détection (qui peut comprendre des circuits linéaires classiques ou à commutation de capacités) ni du contrôleur (qui peut être constitué d'un microprocesseur ou de circuits logiques dédiés). On n'a pas non plus représenté des caractéristiques telles que la protection des entrées à l'encontre des sources à forte énergie telles que les défibrillateurs ou des sources à haute fréquence telles que les bistouris électriques. Ces aspects sont sans incidence sur la présente invention et correspondent à des circuits courants.

## Revendications

1. Un procédé pour ajuster automatiquement un seuil de détection de signaux cardiaques dans un appareil implantable tel que stimulateur cardiaque, cardioverteur ou défibrillateur, comprenant les étapes consistant à :
c) compter (17) une période réfractaire absolue suivant une activité détectée,
d) compter (18, 19) une période réfractaire de bruit suivant cette période réfractaire absolue,
e) redémarrer (21, 25, 19) cette période réfractaire de bruit en cas d'activité détectée dépassant le seuil de détection lors de la période réfractaire de bruit, et
f) incrémenter (24) le seuil de détection en cas de détection (21) dans la période réfractaire de bruit, de manière à accroître le seuil de détection au-dessus de l'amplitude du bruit,
procédé caractérisé en ce que l'étape c) est précédée par les étapes consistant à :
a) mesurer (16) l'amplitude filtrée de l'activité détectée (15), et
b) ajuster (16) le seuil de détection à une fraction prédéterminée de cette amplitude mesurée.

2. Un procédé pour ajuster automatiquement un seuil de détection de signaux cardiaques dans un appareil implantable tel que stimulateur cardiaque, cardioverteur ou défibrillateur, comprenant les étapes consistant à :
― attendre l'apparition d'une activité cardiaque spontanée pendant une période de temps donnée (14),
― en cas d'activité détectée pendant ladite période de temps :
a) mesurer (16) l'amplitude filtrée de l'activité détectée (15), et
b) ajuster (16) le seuil de détection à une fraction prédéterminée de cette amplitude mesurée.
c) compter (17) une période réfractaire absolue suivant une activité détectée,
d) compter (18, 19) une période réfractaire de bruit suivant cette période réfractaire absolue,
e) redémarrer (21, 25, 19) cette période réfractaire de bruit en cas d'activité détectée dépassant le seuil de détection lors de la période réfractaire de bruit, et
f) incrémenter (24) le seuil de détection en cas de détection (21) dans la période réfractaire de bruit, de manière à accroître le seuil de détection au-dessus de l'amplitude du bruit, et
― en l'absence d'activité détectée pendant ladite période de temps et après délivrance d'une impulsion de stimulation,
a) ajuster (30) le seuil de détection à une valeur prédéterminée,
b) compter (31) une période réfractaire absolue suivant l'activité stimulée,
c) compter (32, 19) une période réfractaire de bruit suivant cette période réfractaire absolue,
d ) redémarrer (21, 25, 19) cette période réfractaire de bruit en cas d'activité détectée dépassant le seuil de détection lors de la période réfractaire de bruit, et
e) incrémenter (24) le seuil de détection en cas de détection (21) dans la période réfractaire de bruit, de manière à accroître le seuil de détection au-dessus de l'amplitude du bruit.

3. Le procédé de la revendication 1 ou 2, dans lequel ladite fraction prédéterminée de l'amplitude mesurée se situe entre un huitième et un demi, de préférence un quart.

4. Le procédé de la revendication 3, dans lequel l'étape (16) consistant à ajuster le seuil de détection à une fraction prédéterminée de l'amplitude mesurée comprend l'étape supplémentaire consistant à restreindre ce seuil de détection entre une valeur minimale et une valeur maximale prédéterminées.

5. Le procédé de la revendication 4, dans lequel les valeurs minimale et maximale sont d'environ 0,4 mV et 1,6 mV, respectivement.

6. Le procédé de la revendication 1, comprenant l'étape supplémentaire consistant à omettre (23, 25) l'étape d'incrémentation jusqu'à ce que la période réfractaire de bruit ait été redémarrée un nombre prédéterminé de fois.

7. Le procédé de la revendication 1, comprenant l'étape supplémentaire consistant à omettre l'étape d'incrémentation jusqu'à écoulement d'une durée prédéterminée depuis la fin de la période réfractaire absolue.

8. Le procédé de la revendication 1, comprenant l'étape supplémentaire (28) consistant à incrémenter le seuil de détection en fin de comptage (26) de la période réfractaire de bruit lorsque cette période réfractaire de bruit a été redémarrée un nombre prédéterminé de fois.

9. Le procédé de la revendication 1 ou de la revendication 8, dans lequel l'étape (24 ; 28) consistant à incrémenter le seuil de détection consiste à appliquer un incrément d'environ 0,2 mV.

10. Le procédé de la revendication 1, dans lequel la durée de la période réfractaire de bruit est comprise entre 30 et 100 ms, de préférence environ 31 ms.

## Patentansprüche

1. Ein Verfahren um automatisch eine Detektionsschwelle für Herzsignale in einem implantierbaren Gerät automatisch einzustellen, wie z.B. in einem Herzschrittmacher, Kardioverter oder Defibrillator, das die folgenden Schritte aufweist:
c) Zählen (17) einer absoluten Refraktionsperiode, folgend einer detektierten Aktivität,
d) Zählen (18, 19) einer Refraktionsperiode von Rauschen, folgend dieser absoluten Refraktionsperiode,
e) Wiederingangsetzen (21, 25, 19) dieser Refraktionsperiode von Rauschen im Fall einer detektierten Aktivität, welche die Detektionsschwelle während der Refraktionsperiode von Rauschen übersteigt, und
f) Inkrementieren (24) der Detektionsschwelle im Fall einer Detektion (21) in der Refraktionsperiode von Rauschen, auf eine Weise, um die Detektionsschwelle über die Rauschamplitude zu erhöhen,
wobei das Verfahren dadurch gekennzeichnet ist, daß dem Schritt c) die folgenden Schritte vorhergehen:
a) Messen (16) der gefilterten Amplitude der detektierten Aktivität (15) und
b) Einstellen (16) der Detektionsschwelle auf einen vorbestimmten Bruchteil dieser gemessenen Amplitude.

2. Ein Verfahren um automatisch eine Detektionsschwelle von Herzsignalen in einem implantierbaren Gerät wie z.B. einem Herzschrittmacher, Kardioverter oder Defibrillator einzustellen, das die folgenden Schritte aufweist:
- Abwarten des Auftretens einer spontanen Herzaktivität während einer gegebenen Zeitperiode (14),
- im Fall einer während der Zeitperiode detektierten Aktivität:
a) Messen (16) der gefilterten Amplitude der detektierten Aktivität (15), und
b) Einstellen (16) der Detektionsschwelle auf einen vorbestimmten Bruchteil dieser gemessenen Amplitude.
c) Zählen (17) einer absoluten Refraktionsperiode, folgend einer detektierten Aktivität,
d) Zählen (18, 19) einer Refraktionsperiode von Rauschen, folgend dieser absoluten Refraktionsperiode,
e) Wiederingangsetzen (21, 25, 19) dieser Refraktionsperiode von Rauschen im Fall einer detektierten Aktivität, welche die Detektionsschwelle während der Refraktionsperiode von Rauschen übersteigt, und
f) Inkrementieren (24) der Detektionsschwelle im Fall einer Detektion (21) in der Refraktionsperiode von Rauschen, auf eine Weise, um die Detektionsschwelle über die Amplitude des Rauschens zu erhöhen, und
- bei Nichtvorhandensein einer detektierten Aktivität während der Zeitperiode und nach Lieferung eines Stimulierungsimpulses,
a) Einstellen (30) der Detektionsschwelle auf einen vorbestimmten Wert,
b) Zählen (31) einer absoluten Refraktionsperiode während der stimulierten Aktivität,
c) Zählen (32, 19) einer Refraktionsperiode von Rauschen, folgend dieser absoluten Refraktionsperiode,
d) Wiederingangsetzen (21, 25, 19) dieser Refraktionsperiode von Rauschen im Fall einer detektierten Aktivität, welche die Detektionsschwelle während der Refraktionsperiode von Rauschen übersteigt, und
e) Inkrementieren (24) der Detektionsschwelle im Fall einer Detektion (21) in der Refraktionsperiode von Rauschen, auf eine Weise, um die Detektionsschwelle über die Amplitude des Rauschens zu erhöhen.

3. Verfahren nach Anspruch 1 oder 2, bei welchem der vorbestimmte Bruchteil der gemessenen Amplitude zwischen ein Achtel und ein Halb, vorzugsweise ein Viertel liegt.

4. Verfahren nach Anspruch 3, bei welchem der Schritt 16, bestehend aus Einstellen der Detektionsschwelle auf einen vorbestimmten Bruchteil der gemessenen Amplitude, den zusätzlichen Schritt aufweist, bestehend aus Einschränken dieser Detektionsschwelle zwischen einem minimalen und einem maximalen vorbestimmten Wert.

5. Verfahren nach Anspruch 4, bei welchem der minimale Wert und der maximale Wert ungefähr 0,4mV bzw. 1,6mV sind.

6. Verfahren nach Anspruch 1, welches den zusätzlichen Schritt aufweist, bestehend aus Weglassen (23, 25) des Inkrementierschrittes bis die Refraktionsperiode von Rauschen eine vorbestimmte Anzahl von Malen wieder in Gang gesetzt worden ist.

7. Verfahren nach Anspruch 1, welches den zusätzlichen Schritt aufweist, bestehend aus Weglassen des Inkrementierschrittes bis zum Ablauf einer bestimmten Dauer seit dem Ende der absoluten Refraktionsperiode.

8. Verfahren nach Anspruch 1, welches den zusätzlichen Schritt (28) aufweist, bestehend aus Inkrementieren der Detektionsschwelle am Ende eines Zählens (26) der Refraktionsperiode von Rauschen, wenn diese Refraktionsperiode von Rauschen eine bestimmte Anzahl von Malen wieder in Gang gesetzt wurde.

9. Verfahren nach Anspruch 1 oder nach Anspruch 8, bei welchem der Schritt (24; 28), bestehend aus Inkrementieren der Detektionsschwelle, darin besteht ein Inkrement von ungefähr 0,2mV anzuwenden.

10. Verfahren nach Anspruch 1, bei welchem die Dauer der Refraktionsperiode von Rauschen zwischen 30 und 100ms, vorzugsweise bei ungefähr 31ms liegt.

## Claims

1. A method of automatically adjusting a cardiac signal detection threshold in an implantable device such as a heart stimulator, a cardioverter, or a defibrillator, the method comprising the steps consisting in:
c) timing (17) an absolute refractory period following detected activity;
d) timing (18, 19) a noise refractory period following said absolute refractory period;
e) restarting (21, 25, 19) said noise refractory period in the event of detected activity exceeding the detection threshold during the noise refractory period; and
f) implementing (24) the detection threshold in the event of detection (21) occurring during the noise refractory period, so as to increase the detection threshold to above the amplitude of the noise;
the method being characterized in that step c) is preceded by the steps consisting in:
a) measuring (16) the filtered amplitude of the detected activity (15); and
b) adjusting (16) the detection threshold to a predetermined fraction of said measured amplitude.

2. A method of automatically adjusting a detection threshold for cardiac signals in an implantable device such as a heart stimulator, a cardioverter, or a defibrillator, the method comprising the steps consisting in:
- waiting for the appearance of spontaneous heart activity during a given time period (14);
- in the event of activity being detected during said period of time:
a) measuring (16) the filtered amplitude of the detected activity (15);
b) adjusting (16) the detection threshold to a predetermined fraction of said measured amplitude.
c) timing (17) an absolute refractory period following detected activity;
d) timing (18, 19) a noise refractory period following said absolute refractory period;
e) restarting (21, 25, 19) said noise refractory period in the event of detected activity exceeding the detection threshold during the noise refractory period; and
f) implementing (24) the detection threshold in the event of detection (21) occurring during the noise refractory period, so as to increase the detection threshold to above the amplitude of the noise; and
- in the absence of activity being detected during said period of time and after a stimulation pulse has been delivered:
a) adjusting (30) the detection threshold to a predetermined value;
b) timing (31) an absolute refractory period following the stimulated activity;
c) timing (32, 19) a noise refractory period following said absolute refractory period;
d) restarting (21, 25, 19) said noise refractory period in the event of detected activity exceeding the detection threshold during the noise refractory period; and
e) implementing (24) the detection threshold in the event of detection (21) occurring during the noise refractory period, so as to increase the detection threshold to above the amplitude of the noise.

3. The method of claim 1 or 2, in which said predetermined fraction of the measured amplitude lies between one-eighth and one-half, and is preferably one-fourth.

4. The method of claim 3, in which step (16) consisting in adjusting the detection threshold to a predetermined fraction of the measured amplitude includes the additional step consisting in constraining said detection threshold to lie between predetermined minimum and maximum values.

5. The method of claim 4, in which the minimum and maximum values are respectively about 0.4 mV and about 1.6 mV.

6. The method of claim 1, including the additional step consisting in omitting (23, 25) the incrementation step until the noise refractory period has been restarted some predetermined number of times.

7. The method of claim 1, including the additional step consisting in omitting the incrementation step until a predetermined length of time has elapsed since the end of the absolute refractory period.

8. The method of claim 1, including the additional step (28) consisting in incrementing the detection threshold at the end of measuring (26) the noise refractory period when said noise refractory period has been restarted some predetermined number of times.

9. The method of claim 1 or claim 8, in which the step (24; 28) consisting in incrementing the detection threshold consists in applying an increment of about 0.2 mV.

10. The method of claim 1, in which the duration of the noise refractory period lies in the range 30 ms to 100 ms and is preferably about 31 ms.
